(19) Europäisches Patentamt
European Patent Office
Office européen des brevets

(11) **EP 4 137 312 A1**

(12) **EUROPÄISCHE PATENTANMELDUNG**

(43) Veröffentlichungstag:
**22.02.2023 Patentblatt 2023/08**

(21) Anmeldenummer: **21191535.0**

(22) Anmeldetag: **16.08.2021**

(51) Internationale Patentklassifikation (IPC):
**B32B 18/00** (2006.01)     **C04B 35/486** (2006.01)
**C04B 35/64** (2006.01)     **A61C 13/00** (2006.01)

(52) Gemeinsame Patentklassifikation (CPC):
**C04B 35/64; A61C 5/77; A61C 13/083;**
**B32B 18/00; C04B 35/486;** C04B 2235/3206;
C04B 2235/3208; C04B 2235/3217;
C04B 2235/3224; C04B 2235/3225;
C04B 2235/3229; C04B 2235/3241;
C04B 2235/3262; C04B 2235/3272;
C04B 2235/3275;                    (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Ivoclar Vivadent AG**
**9494 Schaan (LI)**

(72) Erfinder:
 • **Serban, Corina**
  **88131 Lindau (DE)**

 • **Rothbrust, Frank**
  **6822 Röns (AT)**
 • **Ritzberger, Christian**
  **9472 Grabs (CH)**
 • **Krolikowski, Sebastian**
  **8853 Lachen (CH)**

(74) Vertreter: **Uexküll & Stolberg**
**Partnerschaft von**
**Patent- und Rechtsanwälten mbB**
**Beselerstraße 4**
**22607 Hamburg (DE)**

(54) **VERFAHREN ZUR HERSTELLUNG EINER DENTALEN RESTAURATION**

(57) Die Erfindung betrifft ein Verfahren zur Herstellung einer dentalen Restauration, bei dem ein Oxidkeramikmaterial

(a) mindestens einer Wärmebehandlung unterworfen wird bei dem das Oxidkeramikmaterial in Schritt (a) auf eine Temperatur aufgeheizt wird, die im Bereich von 1100 bis 1700°C liegt,

(b) abgekühlt wird,
wobei das Abkühlen
 (b1) einen ersten Abkühlschritt mit der Abkühlrate T1,

 (b2) einen zweiten Abkühlschritt mit der Abkühlrate T2, bei dem der Schritt (b2) in einem Temperaturbereich von 1000 bis 1500°C erfolgt, der Betrag der Abkühlrate T2 weniger als 40 K/min beträgt.
 und
 (b3) einen dritten Abkühlschritt mit der Abkühlrate T3 umfasst, wobei der Betrag der Abkühlrate T2 kleiner als die Beträge der Abkühlraten T1 und T3 ist, bei dem der Betrag der Abkühlrate T1 und der Betrag der Abkühlrate T3 mindestens 40 K/min betragen

EP 4 137 312 A1

(52) Gemeinsame Patentklassifikation (CPC): (Forts.)
C04B 2235/3279; C04B 2235/3281;
C04B 2235/3298; C04B 2235/6562;
C04B 2235/6565; C04B 2235/6567;
C04B 2235/6581; C04B 2235/6585;
C04B 2235/6586; C04B 2235/661;
C04B 2235/765; C04B 2235/9661;
C04B 2237/348; C04B 2237/58

**Beschreibung**

Die vorliegende Erfindung betrifft ein Verfahren, welches ausgehend von einem Oxidkeramikmaterial die Herstellung einer dentalen Restauration mit hervorragenden Eigenschaften in kurzer Zeit ermöglicht. Die Erfindung betrifft auch die Verwendung eines Oxidkeramikmaterials zur Herstellung einer dentalen Restauration mittels des erfindungsgemäßen Verfahrens.

Zur Herstellung vollanatomischer Dentalrestaurationen werden häufig keramische Materialien wie Oxidkeramiken eingesetzt. Diese bieten eine hohe klinische Sicherheit, sind üblicherweise metallfrei, können auch bei minimalinvasiven Präparationen eingesetzt werden und sind preislich im Vergleich zu anderen metallfreien Restaurationen sehr attraktiv. Nachteilig sind jedoch die zahlreichen Arbeitsschritte, die meist zur Herstellung solcher Restaurationen erforderlich sind.

Üblicherweise werden die Restaurationen aus vorgesinterten Rohlingen herausgefräst oder geschliffen, gegebenenfalls eingefärbt, durch thermische Behandlung dichtgesintert und schließlich gegebenenfalls weiter eingefärbt, glasiert und/oder poliert.

Konventionelle Sinterverfahren für Dentalkeramiken beinhalten ein langsames Aufheizen auf eine Maximaltemperatur, bei der das eingesetzte Oxidkeramikmaterial dichtgesintert wird. Bedingt durch die geringe Aufheizgeschwindigkeit dauert ein solcher Sinterprozess typischerweise deutlich mehr als 4 Stunden und trägt dadurch erheblich zu einer vor allem bei Chairside-Behandlungen unbefriedigend großen Dauer des Produktionszyklus von Dentalkeramiken bei.

Ansätze zur Beschleunigung des Sinterprozesses durch Erhöhung der Aufheizgeschwindigkeit sind grundsätzlich bekannt.

So beschreibt EP 2 098 188 A1 einen Dentalofen und ein Verfahren zum Sintern von Dentalmaterialien, bei dem der Ofen in einer ersten Aufheizperiode mit einer schnellen Aufheizrate von mehr als 50 K/min auf eine Vorsintertemperatur von mindestens 1000°C aufgeheizt wird.

EP 2 101 133 A1 beschreibt einen Sinterofen und ein Verfahren zum Sintern von Dentalpräparaten, bei dem die Dentalpräparate entlang einer Sinterstrecke bewegt und dabei verschiedenen Temperaturen ausgesetzt werden. Dabei können in einem ersten Abschnitt hohe Aufheizraten von 300 K/min oder mehr eingesetzt werden.

WO 2012/057829 A2 beschreibt ein Verfahren zum schnellen Sintern von Keramik unter Verwendung von elektromagnetischer Induktion oder eines Plasmas.

WO 2015/091744 A1 beschreibt ein Verfahren zur Planung einer Sinterung eines Zahnersatzteils, bei dem in Abhängigkeit von bestimmten Geometrie- und Materialparametern des herzustellenden Zahnersatzteils ein Temperaturprofil für die Wärmebehandlung des Zahnersatzteils automatisch mittels eines Computers bestimmt wird. Dabei wird für die Sinterung bestimmter Zahnersatzteile eine Heizrate zwischen 100 K/min und 400 K/min verwendet.

WO 2015/121364 A1 beschreibt einen Sinterofen für Dentalbauteile mit einer Aufheizvorrichtung, die im Nutzbereich eine Aufheizrate von mindestens 200 K/min ermöglicht.

Auch die Sinterung von Dentalmaterialien unter Schutzgas oder im Vakuum ist bekannt.

WO 2011/020688 A1 beschreibt eine Vorrichtung zum sauerstofffreien Sintern von Metall oder Keramik in der Dentaltechnik unter Schutzgas.

EP 2 703 760 A1 beschreibt einen Dentalofen zum Sintern eines Zahnersatzes, dessen Heizraum entweder mittels einer konventionellen Verschlussvorrichtung wie etwa einer Tür oder mittels eines Aufsatzes mit einem Vakuumbehälter verschließbar ist und dadurch wahlweise zum normalen Sintern oder zum Vakuumsintern eingesetzt werden kann.

WO 2017/189414 A1 beschreibt ein Verfahren zur Herstellung einer Dentalrestoration, welches eine zusätzliche Wärmebehandlung zur Herbeiführung einer Farbänderung umfasst.

EP 3 659 548 A1 beschreibt ein Verfahren zur Herstellung einer dentalen Restauration, bei dem ein Oxidkeramikmaterial mindestens zwei Wärmebehandlungen unterworfen wird, wobei die erste Wärmebehandlung bei niedrigerem Druck als die zweite Wärmebehandlung erfolgt.

Es hat sich jedoch gezeigt, dass die bekannten Ansätze zur Beschleunigung des Sinterprozesses zu Keramikmaterialien führen, deren Eigenschaften insbesondere in optischer Hinsicht den hohen Anforderungen im Dentalbereich nicht genügen.

Der Erfindung liegt daher die Aufgabe zugrunde, ein Verfahren zur Herstellung einer dentalen Restauration bereitzustellen, mit dem in kurzer Zeit durch Sintern von Oxidkeramikmaterial dentale Restaurationen mit hervorragenden mechanischen und insbesondere optischen Eigenschaften hergestellt werden können.

Diese Aufgabe wird erfindungsgemäß durch das Verfahren zur Herstellung einer dentalen Restauration nach den Ansprüchen 1 bis 21 gelöst. Gegenstand der Erfindung ist auch die Verwendung eines Oxidkeramikmaterials zur Herstellung einer dentalen Restauration nach Anspruch 22.

Das erfindungsgemäße Verfahren zur Herstellung einer dentalen Restauration zeichnet sich dadurch aus, dass ein Oxidkeramikmaterial

(a) mindestens einer Wärmebehandlung unterworfen wird und
(b) abgekühlt wird,

wobei das Abkühlen

(b1) einen ersten Abkühlschritt mit der Abkühlrate T1 und
(b2) einen zweiten Abkühlschritt mit der Abkühlrate T2

umfasst und wobei der Betrag der Abkühlrate T2 kleiner als der Betrag der Abkühlrate T1 ist.

Es wurde überraschend gefunden, dass das erfindungsgemäße Verfahren eine sehr schnelle Sinterung von Oxidkeramiken zu dentalen Restaurationen ermöglicht, die gute mechanische Eigenschaften und insbesondere eine hohe Dichte aufweisen und zugleich auch in ästhetischer Hinsicht die hohen Anforderungen an dentale Restaurationen erfüllen und die optischen Eigenschaften von natürlichem Zahnmaterial ausgezeichnet imitieren können.

Bei dem in Schritt (a) eingesetzten Oxidkeramikmaterial handelt es sich üblicherweise um ein nicht dichtgesintertes und insbesondere um ein vorgesintertes Oxidkeramikmaterial. Üblicherweise weist das eingesetzte Oxidkeramikmaterial eine relative Dichte im Bereich von 30 bis 90%, insbesondere im Bereich von 40 bis 80% und bevorzugt im Bereich von 50 bis 70%, jeweils bezogen auf die Reindichte des Oxidkeramikmaterials, auf.

Die relative Dichte ist dabei das Verhältnis der scheinbaren Dichte des Oxidkeramikmaterials zur Reindichte des Oxidkeramikmaterials.

Die scheinbare Dichte des Oxidkeramikmaterials kann bestimmt werden, indem dieses gewogen und sein Volumen geometrisch ermittelt wird. Die Dichte wird dann nach der bekannten Formel

$$\text{Dichte} = \text{Masse} \; / \; \text{Volumen}$$

berechnet.

Die Bestimmung der Reindichte des Oxidkeramikmaterials erfolgt, indem das Oxidkeramikmaterial auf ein Pulver mit einer mittleren Teilchengröße von 10 bis 30 $\mu$m, insbesondere von 20 $\mu$m, bezogen auf die Anzahl der Teilchen, gemahlen und die Dichte des Pulvers mittels Pyknometer ermittelt wird. Die Bestimmung der Teilchengröße kann beispielsweise mit dem CILAS® Particle Size Analyzer 1064 der Firma Quantachrome GmbH & Co. KG mittels Laserbeugung nach ISO 13320 (2009) durchgeführt werden.

In einer bevorzugten Ausführungsform des Verfahrens umfasst das Abkühlen

(b1) einen ersten Abkühlschritt mit der Abkühlrate T1,
(b2) einen zweiten Abkühlschritt mit der Abkühlrate T2 und
(b3) einen dritten Abkühlschritt mit der Abkühlrate T3,

wobei der Betrag der Abkühlrate T2 kleiner als die Beträge der Abkühlraten T1 und T3 ist.

Weiterhin ist es erfindungsgemäß bevorzugt, dass der Schritt (b2), in dem der Betrag der Abkühlrate T2 kleiner als die Beträge der Abkühlrate T1 und gegebenenfalls der Abkühlrate T3 ist, in einem Temperaturbereich von 1000 bis 1500°C, bevorzugt 1100 bis 1400°C und besonders bevorzugt 1200 bis 1300°C erfolgt.

Es ist außerdem bevorzugt, dass in Schritt (b2) der Betrag der Abkühlrate T2 weniger als 60 K/min, bevorzugt weniger als 50 K/min, besonders bevorzugt weniger als 40 K/min, weiter bevorzugt weniger als 25 K/min, ganz besonders bevorzugt weniger als 10 K/min und am meisten bevorzugt weniger als 5 K/min beträgt. In einer besonders bevorzugten Ausführungsform des Verfahrens erfolgt Schritt (b2) bei im Wesentlichen konstanter Temperatur, so dass die Abkühlrate T2 etwa 0 K/min beträgt.

Schritt (b2) kann insbesondere für eine Dauer von 1 bis 20 min, bevorzugt 1 bis 10 min, weiter bevorzugt 2 bis 8 min, besonders bevorzugt 3 bis 7 min und ganz besonders bevorzugt 4 bis 6 min durchgeführt werden.

Erfindungsgemäß ist der Betrag der Abkühlrate T2 kleiner als die Beträge der Abkühlrate T1 und gegebenenfalls der Abkühlrate T3. In einer bevorzugten Ausführungsform beträgt der Betrag der Abkühlrate T1 mindestens 40 K/min, bevorzugt mindestens 50 K/min und besonders bevorzugt mindestens 60 K/min und liegt insbesondere im Bereich von 40 bis 200 K/min, bevorzugt 50 bis 100 K/min und besonders bevorzugt 60 bis 80 K/min. In einer anderen bevorzugten Ausführungsform beträgt der Betrag der Abkühlrate T3 mindestens 40 K/min, bevorzugt mindestens 50 K/min und besonders bevorzugt mindestens 60 K/min und liegt insbesondere im Bereich von 40 bis 200 K/min, bevorzugt 50 bis 100 K/min und besonders bevorzugt 60 bis 80 K/min. In einer besonders bevorzugten Ausführungsform betragen die Beträge der Abkühlraten T1 und T3 jeweils mindestens 40 K/min, bevorzugt mindestens 50 K/min und besonders bevorzugt mindestens 60 K/min und liegen insbesondere im Bereich von 40 bis 200 K/min, bevorzugt 50 bis 100 K/min und besonders bevorzugt 60 bis 80 K/min.

In Schritt (a) wird das Oxidkeramikmaterial vorzugsweise auf eine Temperatur aufgeheizt, die im Bereich von 1100 bis 1700°C, bevorzugt im Bereich von 1300 bis 1600°C, weiter bevorzugt im Bereich von 1400 bis 1550°C und besonders bevorzugt im Bereich von 1450 bis 1500°C liegt und am meisten bevorzugt etwa 1480°C beträgt. Es ist weiter bevorzugt, dass das Oxidkeramikmaterial nach der Durchführung von Schritt (a) eine relative Dichte im Bereich von 90 bis 97%, bevorzugt im Bereich von 93 bis 96% und besonders bevorzugt eine relative Dichte von etwa 95%, jeweils bezogen auf die Reindichte des Oxidkeramikmaterials, aufweist.

Vorzugsweise wird das Oxidkeramikmaterial in Schritt (a) mit einer Heizrate im Bereich von 5 bis 500 K/min, bevorzugt 50 bis 250 K/min und besonders bevorzugt 100 bis 200 K/min aufgeheizt. In einer bevorzugten Ausführungsform wird das Oxidkeramikmaterial zunächst mit einer Heizrate von 50 bis 500 K/min, bevorzugt 75 bis 250 K/min und besonders bevorzugt 100 bis 200 K/min auf eine Temperatur aufgeheizt, die 100 bis 800 K, bevorzugt 300 bis 700 K und besonders bevorzugt 500 bis 600 K unterhalb der maximalen in Schritt (a) erreichten Temperatur liegt, und anschließend mit einer Heizrate von 5 bis 200 K/min, bevorzugt 10 bis 100 K/min und besonders bevorzugt 25 bis 50 K/min weiter aufgeheizt.

In Schritt (b) wird das Oxidkeramikmaterial vorzugsweise auf eine Temperatur abgekühlt, die im Bereich von 20 bis 1300°C, bevorzugt im Bereich von 100 bis 1250°C und besonders bevorzugt im Bereich von 1000 bis 1200°C liegt. Nach Erreichen einer solchen Temperatur kann das Oxidkeramikmaterial aus der Heizkammer entfernt werden.

Gemäß einer weiteren Ausführungsform ist ein Verfahren besonders bevorzugt, bei dem das Oxidkeramikmaterial in Schritt (a)

    (a1) einer ersten Wärmebehandlung unterworfen wird und
    (a2) einer zweiten Wärmebehandlung unterworfen wird,

wobei die Wärmebehandlung in Schritt (a1) bei niedrigerem Druck als die Wärmebehandlung in Schritt (a2) erfolgt.

Bevorzugt erfolgt die Wärmebehandlung in Schritt (a1) bei einem Druck von weniger als 200 mbar, vorzugsweise weniger als 100 mbar und besonders bevorzugt weniger als 50 mbar, und insbesondere bei einem Druck im Bereich von 0,1 bis 200 mbar, vorzugsweise im Bereich von 1 bis 150 mbar und besonders bevorzugt im Bereich von 50 bis 100 mbar.

Die Einstellung dieses Drucks kann bei Umgebungstemperatur erfolgen, bevor mit dem Aufheizen des Oxidkeramikmaterials begonnen wird. Alternativ kann das Oxidkeramikmaterial zunächst auf eine oberhalb der Umgebungstemperatur liegende Temperatur aufgeheizt werden, bevor der für Schritt (a) definierte Druck eingestellt wird. Diese Temperatur liegt vorzugsweise im Bereich von 20 bis 500°C und insbesondere im Bereich von 25 bis 100°C.

In Schritt (a2) wird das Oxidkeramikmaterial vorzugsweise weiter aufgeheizt und bei einer, vorzugsweise konstanten, Temperatur im Bereich von 1100 bis 1700°C, insbesondere im Bereich von 1300 bis 1600°C, bevorzugt im Bereich von 1400 bis 1550°C, bevorzugt im Bereich von 1450 bis 1500°C und am meisten bevorzugt bei einer Temperatur etwa 1480°C gehalten und gesintert. Dabei erfolgt das weitere Aufheizen vorzugsweise mit einer Heizrate von 5 bis 200 K/min, insbesondere 10 bis 100 K/min und bevorzugt 25 bis 50 K/min. Das Halten erfolgt vorzugsweise für 1 bis 60 Minuten, insbesondere 5 bis 30 Minuten, bevorzugt 10 bis 25 Minuten und besonders bevorzugt 15 bis 20 Minuten. Durch das Halten bei der entsprechenden Temperatur wird das Oxidkeramikmaterial typischerweise dichtgesintert. Danach weist das Oxidkeramikmaterial vorzugsweise eine relative Dichte von mindestens 97%, insbesondere mindestens 98%, bevorzugt mindestens 99% und am meisten bevorzugt mindestens 99,5%, jeweils bezogen auf die Reindichte des Oxidkeramikmaterials, auf.

Die Wärmebehandlung in Schritt (a2) wird vorzugsweise bei einem Druck von mehr als 500 mbar und insbesondere bei Umgebungsdruck ausgeführt.

Bevorzugt erfolgt die Wärmebehandlung in Schritt (a2) in einer sauerstoffhaltigen Atmosphäre. Als sauerstoffhaltige Atmosphäre kommen insbesondere Luft, mit Sauerstoff angereicherte Luft sowie Sauerstoff in Frage. Zur Einstellung einer solchen Atmosphäre kann die für die Wärmebehandlung verwendete Heizkammer mit Luft und/oder Sauerstoff gefüllt werden. In einer bevorzugten Ausführungsform wird die für die Wärmebehandlung verwendete Heizkammer während Schritt (a2) diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt, insbesondere mit einer Strömungsgeschwindigkeit von 0,1 bis 50 l/min, bevorzugt 1 bis 10 l/min und besonders bevorzugt 2 bis 5 l/min.

Außerdem ist es bevorzugt, dass das Oxidkeramikmaterial in Schritt (a1) auf eine Temperatur aufgeheizt wird, die 0 bis 500 K, insbesondere 10 bis 250 K, bevorzugt 50 bis 150 K und besonders bevorzugt 75 bis 100 K unterhalb der Temperatur oder des Temperaturbereichs liegt, bei der/dem das Oxidkeramikmaterial in Schritt (a2) gehalten wird.

Das nach dem erfindungsgemäßen Verfahren erhaltene Oxidkeramikmaterial weist vorzugsweise eine zahlenmittlere Korngröße im Bereich von 1 nm bis 1000 nm, insbesondere von 10 nm bis 800 nm und bevorzugt von 100 nm bis 600 nm auf. Die zahlenmittlere Korngröße kann insbesondere nach dem Linienschnittverfahren gemäß DIN EN 623-3 oder ASTM E 112 bestimmt werden, wobei der ermittelte Wert zur Umrechnung auf die reale zahlenmittlere Korngröße im dreidimensionalen Mikrogefüge nach M. I. Mendelson, J. Am. Ceram. Soc. 1969, 52(8), 443-446 mit einer Proportionalitätskonstante von 1,56 multipliziert wird.

Das erfindungsgemäße Verfahren eignet sich für verschiedene Arten von Oxidkeramikmaterialien. Oxidkeramikmaterialien sind allgemein hochkristalline Keramikmaterialien, die auf Oxidverbindungen basieren und allenfalls einen sehr geringen Anteil an Glasphase aufweisen. Typische Oxidkeramikmaterialien basieren auf $ZrO_2$, $Al_2O_3$, $TiO_2$, MgO, Kombinationen, Mischkristallen oder Kompositen davon, insbesondere $ZrO_2/Al_2O_3$ (ZTA), $Al_2O_3/ZrO_2$ (ATZ) oder $ZrO_2$/Spinell, wobei Spinell vorzugsweise Sr-Spinell, Mg-Spinell, La-Spinell und/oder Ce-Spinell ist. Oxidkeramikmaterialien auf Basis von $ZrO_2$ und/oder $Al_2O_3$ sind erfindungsgemäß bevorzugt.

Besonders bevorzugt sind Oxidkeramikmaterialien auf Basis von Zirkonoxid und insbesondere auf Basis von polykristallinem tetragonalem Zirkonoxid (TZP). Ganz besonders bevorzugt sind Oxidkeramikmaterialien auf Basis von Zirkonoxid, bei denen das Zirkonoxid mit $Y_2O_3$, $La_2O_3$, $CeO_2$, MgO und/oder CaO stabilisiert ist und vorzugsweise mit 2 bis 12 mol-%, insbesondere 3 bis 6 mol-%, dieser Oxide, bezogen auf den Gehalt an Zirkonoxid, stabilisiert ist.

Es ist weiter bevorzugt, dass das Oxidkeramikmaterial eingefärbt ist. Hierunter wird erfindungsgemäß ein Oxidkeramikmaterial verstanden, das mit einem oder mehreren färbenden Elementen versetzt ist. Beispiele für geeignete färbende Elemente sind Fe, Mn, Cr, Pr, Tb, Er, Yb, Ce, Co, Ni, Nd, Cu und Bi. Bevorzugt enthält das Oxidkeramikmaterial insbesondere Fe. Besonders bevorzugt umfasst das Oxidkeramikmaterial mindestens zwei Schichten, die sich insbesondere in ihrer Farbe unterscheiden.

Im Sinne der vorliegenden Anmeldung beziehen sich die Begriffe "Farbe" und "eingefärbt" auf die Farbe, Helligkeit und/oder Transluzenz eines Materials.

"Transluzenz" ist die Lichtdurchlässigkeit eines Materials, Körpers oder einer Schicht, d.h. das Verhältnis von durchgelassener zu eingestrahlter Lichtintensität.

Farben können auch durch die Farbkoordinaten L*, a* und b* im L*a*b*-Farbraum oder durch einen in der Dentalindustrie üblicherweise verwendeten Farbcode charakterisiert werden.

Im L*a*b*-Farbraum beschreibt der Wert $L^*$ die Helligkeit einer Farbe mit Werten von 0 (schwarz) bis 100 (weiß), der Wert $a^*$ den Grün- oder Rotanteil einer Farbe, wobei negative Werte für Grün und positive Werte für Rot stehen, und der Wert b* den Blau- oder Gelbanteil einer Farbe, wobei negative Werte für Blau und positive Werte für Gelb stehen. Farbunterschiede können im L*a*b*-Farbraum durch den $\Delta E^*$-Wert ausgedrückt werden, der wie folgt berechnet wird:

$$\Delta E^* = \sqrt{(\Delta L^*)^2 + (\Delta a^*)^2 + (\Delta b^*)^2}.$$

Beispiele für in der Dentalindustrie übliche Farbcodes sind Vitapan classical® und Vita 3D Master®, beide von der VITA Zahnfabrik H. Rauter GmbH & Co. KG, und Chromascop® von der Ivoclar Vivadent AG. Die Transluzenz kann durch den Kontrastwert CR charakterisiert werden, wobei 0% vollständig transparent und 100% vollständig opak bedeutet.

Üblicherweise erfolgen die Bestimmung der Farbkoordinaten $L^*$, $a^*$ und $b^*$ nach DIN 5033 und DIN 6174 und die Bestimmung der Transluzenz nach BS 5612. Die entsprechenden Messungen können insbesondere mittels eines Spektrophotometers vom Typ CM-3700d (Konica-Minolta) durchgeführt werden. Hierzu werden für die Messungen Probenkörper eingesetzt, die mit Diamantpartikeln (Partikelgröße 15-20 $\mu$m) beidseitig nass beschliffen wurden, um eine finale Probendicke von 2,00 $\pm$ 0,025 mm zu erhalten.

Vorzugsweise liegen die Farbe oder die Farben der erfindungsgemäß erhaltenen dentalen Restauration im Bereich der Farben natürlicher Zähne. Besonders bevorzugt weisen die erfindungsgemäß erhaltenen dentalen Restauration einen L*-Wert im Bereich von 50 bis 100, insbesondere im Bereich von 80 bis 97, einen a*-Wert im Bereich von -10 bis 10, insbesondere im Bereich von -1 bis 5, einen b*-Wert im Bereich von 0 bis 50, insbesondere im Bereich von 1 bis 20, und/oder einen CR-Wert im Bereich von 50 bis 100%, insbesondere im Bereich von 75 bis 99%, auf.

Das erfindungsgemäße Verfahren eignet sich in besonderer Weise für die Herstellung von dentalen Restaurationen. Besonders bevorzugte dentale Restaurationen sind Brücken, Inlays, Onlays, Kronen, Veneers, Schalen und Abutments. Ganz besonders eignet sich das erfindungsgemäße Verfahren zur Herstellung von dentalen Restaurationen, insbesondere Brücken, die zwei oder mehr Glieder umfassen.

Die Erfindung betrifft auch die Verwendung eines Oxidkeramikmaterials zur Herstellung einer dentalen Restauration, bei der das Oxidkeramikmaterial

(a) mindestens einer Wärmebehandlung bei einer Temperatur von mindestens 1000°C unterworfen wird und
(b) abgekühlt wird,

wobei das Abkühlen

(b1) einen ersten Abkühlschritt mit der Abkühlrate T1 und

(b2) einen zweiten Abkühlschritt mit der Abkühlrate T2

umfasst und wobei der Betrag der Abkühlrate T2 kleiner als der Betrag der Abkühlrate T1 ist.

Bevorzugte Ausführungsformen der Verwendung sind wie oben für das erfindungsgemäße Verfahren beschrieben.

Die Erfindung wird im Folgenden anhand von Beispielen näher erläutert.

**Beispiele**

**Beispiele 1A bis 1F**

Aus einem kommerziellen Oxidkeramikmaterial auf Basis von Zirkonoxid mit 9,23 Gew.-% $Y_2O_3$, 0,045 Gew.-% $Al_2O_3$ und 0,25 Gew.-% $Fe_2O_3$ (Zpex smile yellow der Firma Tosoh) wurden durch uniaxiales Pressen mit einem Druck von 150 MPa und Wärmebehandlung bei 1000°C für 2 Stunden Prüfkörper mit einem Durchmesser von 24 mm und einer Höhe von 2,9 mm hergestellt.

Die Prüfkörper wurden in einem Sinterofen mit $MoSi_2$-Heizelement gesintert. Dazu wurde der Prüfkörper bei Raumtemperatur in die Heizkammer des Sinterofens eingebracht, die Heizkammer verschlossen und in der Heizkammer ein partielles Vakuum mit einem finalen Druck von etwa 50 bis 100 mbar erzeugt. Der Prüfkörper wurde mit einer Heizrate von etwa 130 K/min auf eine Temperatur von etwa 900°C, danach mit einer Heizrate von etwa 50 K/min auf eine Temperatur von etwa 1220°C und weiter mit einer Heizrate von etwa 10 K/min auf eine Temperatur von etwa 1400°C aufgeheizt. Bei Erreichen dieser Temperatur wurde die Heizkammer mit Frischluft geflutet und anschließend kontinuierlich mit einer Strömungsgeschwindigkeit von etwa 2,2 l/min mit Frischluft durchströmt, während der Prüfkörper mit einer Heizrate von etwa 10 K/min weiter auf eine Temperatur von 1480°C aufgeheizt und bei dieser Temperatur etwa 17 Minuten lang gehalten wurde. Danach wurde der Prüfkörper gemäß Tabelle 1 mit einer Abkühlrate von etwa 70 K/min auf eine Temperatur T abgekühlt, für eine Dauer von t Minuten bei dieser Temperatur gehalten und dann mit einer Abkühlrate von etwa 70 K/min auf eine Temperatur von etwa 1200°C weiter abgekühlt. Anschließend wurde die Heizkammer geöffnet. Die Gesamtdauer des Sinterprozesses betrug zwischen 64 und 66 min.

**Beispiel 1G (Vergleich)**

Beispiel 1A wurde wiederholt, wobei jedoch der Prüfkörper mit einer durchgehenden Abkühlrate ohne Unterbrechung von etwa 70 K/min von 1480°C auf 1200°C abgekühlt wurde. Die Gesamtdauer des Sinterprozesses betrug etwa 60 min.

**Beispiel 1H (Vergleich)**

Beispiel 1A wurde wiederholt, wobei jedoch ein langsamer Sinterprozess verwendet wurde. Dazu wurde der Prüfkörper mit einer Heizrate von etwa 10 K/min auf eine Temperatur von etwa 900°C aufgeheizt, bei dieser Temperatur für 30 min gehalten, weiter mit einer Heizrate von etwa 3 K/min auf eine Temperatur von etwa 1500°C aufgeheizt und bei dieser Temperatur etwa 120 min gehalten. Danach wurde der Prüfkörper mit einer Abkühlrate von etwa 10 K/min auf 900°C und weiter mit einer Abkühlrate von etwa 8 K/min auf 300°C abgekühlt. Anschließend wurde die Heizkammer geöffnet. Die Gesamtdauer des Sinterprozesses betrug etwa 575 min.

Die CR-Werte und Farbkoordinaten der in den Beispielen 1A-H erhaltenen Oxidkeramikmaterialien sind in Tabelle 1 wiedergegeben. Man erkennt, dass die erfindungsgemäßen Beispiele 1A bis 1F im Vergleich zum ebenfalls mit einem schnellen Sinterprozess erhaltenen Beispiel 1G deutlich höhere a*-Werte und b*-Werte zeigen und die a*-Werte durchgängig im positiven Bereich liegen. Dadurch sind diese Beispiele besser geeignet, die Farbeigenschaften von natürlichem Zahnmaterial zu imitieren. Zugleich benötigt der für die erfindungsgemäßen Beispiele 1A bis 1F verwendete Sinterprozess nur etwa ein Zehntel der Dauer des langsamen Sinterprozesses gemäß Beispiel 1H.

Tabelle 1

| Beispiel | Sinterdauer [min] | Vakuum bis [°C] | Abkühlung | | CR [%] | L* | a* | b* |
|---|---|---|---|---|---|---|---|---|
| | | | T [°C] | t [min] | | | | |
| 1A | 64 | 1400 | 1200 | 4 | 98,84 | 71,46 | 0,74 | 18,44 |
| 1B | 66 | 1400 | 1200 | 6 | 99,80 | 75,48 | 0,70 | 19,31 |
| 1C | 64 | 1400 | 1250 | 4 | 98,99 | 71,85 | 0,82 | 18,68 |

(fortgesetzt)

| Beispiel | Sinterdauer [min] | Vakuum bis [°C] | Abkühlung | | CR [%] | L* | a* | b* |
|---|---|---|---|---|---|---|---|---|
| | | | T [°C] | t [min] | | | | |
| 1D | 66 | 1400 | 1250 | 6 | 99,29 | 75,08 | 1,14 | 19,49 |
| 1E | 64 | 1400 | 1300 | 4 | 98,21 | 71,03 | 1,18 | 19,58 |
| 1F | 66 | 1400 | 1300 | 6 | 99,79 | 74,80 | 0,63 | 19,14 |
| 1G* | 60 | 1400 | - | - | 99,32 | 74,05 | -0,60 | 16,31 |
| 1H* | 575 | - | - | - | 98,60 | 77,98 | 3,03 | 20,74 |
| * (Vergleich) | | | | | | | | |

**Patentansprüche**

1. Verfahren zur Herstellung einer dentalen Restauration, bei dem ein Oxidkeramikmaterial

   (a) mindestens einer Wärmebehandlung unterworfen wird und
   (b) abgekühlt wird,

   wobei das Abkühlen

   (b1) einen ersten Abkühlschritt mit der Abkühlrate T1 und
   (b2) einen zweiten Abkühlschritt mit der Abkühlrate T2

   umfasst und wobei der Betrag der Abkühlrate T2 kleiner als der Betrag der Abkühlrate T1 ist.

2. Verfahren nach Anspruch 1, bei dem das Abkühlen

   (b1) einen ersten Abkühlschritt mit der Abkühlrate T1,
   (b2) einen zweiten Abkühlschritt mit der Abkühlrate T2 und
   (b3) einen dritten Abkühlschritt mit der Abkühlrate T3

   umfasst, wobei der Betrag der Abkühlrate T2 kleiner als die Beträge der Abkühlraten T1 und T3 ist.

3. Verfahren nach Anspruch 1 oder 2, bei dem der Schritt (b2) in einem Temperaturbereich von 1000 bis 1500°C, bevorzugt 1100 bis 1400°C und besonders bevorzugt 1200 bis 1300°C erfolgt.

4. Verfahren nach einem der Ansprüche 1 bis 3, bei dem in Schritt (b2) der Betrag der Abkühlrate T2 weniger als 60 K/min, bevorzugt weniger als 50 K/min, besonders bevorzugt weniger als 40 K/min, weiter bevorzugt weniger als 25 K/min, ganz besonders bevorzugt weniger als 10 K/min und am meisten bevorzugt weniger als 5 K/min beträgt.

5. Verfahren nach einem der Ansprüche 1 bis 4, bei dem Schritt (b2) bei im Wesentlichen konstanter Temperatur erfolgt.

6. Verfahren nach einem der Ansprüche 1 bis 5, bei dem der Schritt (b2) für eine Dauer von 1 bis 20 min, bevorzugt 1 bis 10 min, weiter bevorzugt 2 bis 8 min, besonders bevorzugt 3 bis 7 min und ganz besonders bevorzugt 4 bis 6 min durchgeführt wird.

7. Verfahren nach einem der Ansprüche 1 bis 6, bei dem der Betrag der Abkühlrate T1 und/oder der Betrag der Abkühlrate T3 mindestens 40 K/min, bevorzugt mindestens 50 K/min und besonders bevorzugt mindestens 60 K/min beträgt und insbesondere im Bereich von 40 bis 200 K/min, bevorzugt 50 bis 100 K/min und besonders bevorzugt 60 bis 80 K/min liegt.

8. Verfahren nach einem der Ansprüche 1 bis 7, bei dem das Oxidkeramikmaterial in Schritt (a) auf eine Temperatur aufgeheizt wird, die im Bereich von 1100 bis 1700°C, bevorzugt im Bereich von 1300 bis 1600°C, weiter bevorzugt im Bereich von 1400 bis 1550°C und besonders bevorzugt im Bereich von 1450 bis 1500°C liegt und am meisten bevorzugt etwa 1480°C beträgt.

9. Verfahren nach einem der Ansprüche 1 bis 8, bei dem das Oxidkeramikmaterial in Schritt (a) mit einer Heizrate im Bereich von 5 bis 500 K/min, bevorzugt 50 bis 250 K/min und besonders bevorzugt 100 bis 200 K/min aufgeheizt wird.

10. Verfahren nach einem der Ansprüche 1 bis 9, bei dem das Oxidkeramikmaterial in Schritt (b) auf eine Temperatur abgekühlt wird, die im Bereich von 20 bis 1300°C, bevorzugt im Bereich von 100 bis 1250°C und besonders bevorzugt im Bereich von 1000 bis 1200°C liegt.

11. Verfahren nach einem der Ansprüche 1 bis 10, bei dem das Oxidkeramikmaterial in Schritt (a)

   (a1) einer ersten Wärmebehandlung unterworfen wird und
   (a2) einer zweiten Wärmebehandlung unterworfen wird,

wobei die Wärmebehandlung in Schritt (a1) bei niedrigerem Druck als die Wärmebehandlung in Schritt (a2) erfolgt.

12. Verfahren nach Anspruch 11, bei dem die Wärmebehandlung in Schritt (a1) bei einem Druck von weniger als 200 mbar, vorzugsweise weniger als 100 mbar und besonders bevorzugt weniger als 50 mbar und insbesondere bei einem Druck im Bereich von 0,1 bis 200 mbar, vorzugsweise im Bereich von 1 bis 150 mbar und besonders bevorzugt im Bereich von 50 bis 100 mbar erfolgt.

13. Verfahren nach Anspruch 11 oder 12, bei dem das Oxidkeramikmaterial in Schritt (a2) weiter aufgeheizt wird und bei einer, vorzugsweise konstanten, Temperatur im Bereich von 1100 bis 1700°C, insbesondere im Bereich von 1300 bis 1600°C, bevorzugt im Bereich von 1400 bis 1550°C, besonders bevorzugt im Bereich von 1450 bis 1500°C und am meisten bevorzugt bei einer Temperatur von etwa 1480°C gehalten und gesintert wird, wobei das Halten vorzugsweise für 1 bis 60 Minuten, bevorzugt 5 bis 30 Minuten, weiter bevorzugt 10 bis 25 Minuten und besonders bevorzugt 15 bis 20 Minuten erfolgt.

14. Verfahren nach einem der Ansprüche 11 bis 13, bei dem die Wärmebehandlung in Schritt (a2) bei einem Druck von mehr als 500 mbar und insbesondere bei Umgebungsdruck und/oder in einer sauerstoffhaltigen Atmosphäre und insbesondere in Luft, mit Sauerstoff angereicherter Luft oder Sauerstoff erfolgt.

15. Verfahren nach Anspruch 14, bei dem während Schritt (a2) die Heizkammer diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt wird, insbesondere mit einer Strömungsgeschwindigkeit von 0,1 bis 50 1/min, bevorzugt 1 bis 10 l/min und besonders bevorzugt 2 bis 5 1/min.

16. Verfahren nach einem der Ansprüche 11 bis 15, bei dem das Oxidkeramikmaterial in Schritt (a1) auf eine Temperatur aufgeheizt wird, die 0 bis 500 K, insbesondere 10 bis 250 K, bevorzugt 50 bis 150 K und besonders bevorzugt 75 bis 100 K unterhalb der Temperatur oder des Temperaturbereichs liegt, bei der/dem das Oxidkeramikmaterial in Schritt (a2) gehalten wird.

17. Verfahren nach einem der Ansprüche 1 bis 16, bei dem das Oxidkeramikmaterial auf Zirkonoxid und insbesondere auf polykristallinem tetragonalen Zirkonoxid (TZP) basiert.

18. Verfahren nach Anspruch 17, bei dem das Zirkonoxid mit $Y_2O_3$, $CeO_2$, MgO und/oder CaO stabilisiert ist und vorzugsweise mit 2 bis 12 mol-%, insbesondere 3 bis 6 mol-%, dieser Oxide, bezogen auf den Gehalt an Zirkonoxid, stabilisiert ist.

19. Verfahren nach einem der Ansprüche 1 bis 18, bei dem das Oxidkeramikmaterial eingefärbt ist und vorzugsweise mindestens zwei Schichten umfasst, die sich insbesondere in ihrer Farbe unterscheiden.

20. Verfahren nach einem der Ansprüche 1 bis 19, bei dem das Oxidkeramikmaterial mindestens ein färbendes Element ausgewählt aus der Gruppe Fe, Mn, Cr, Pr, Tb, Er, Yb, Ce, Co, Ni, Nd, Cu und Bi und insbesondere Fe enthält.

21. Verfahren nach einem der Ansprüche 1 bis 20, bei dem die dentale Restauration eine Brücke, ein Inlay, ein Onlay, eine Krone, ein Veneer, eine Schale oder ein Abutment ist und vorzugsweise zwei oder mehr Glieder umfasst.

22. Verwendung eines Oxidkeramikmaterials zur Herstellung einer dentalen Restauration, bei der das Oxidkeramikmaterial

   (a) mindestens einer Wärmebehandlung unterworfen wird und
   (b) abgekühlt wird,

wobei das Abkühlen

   (b1) einen ersten Abkühlschritt mit der Abkühlrate T1 und
   (b2) einen zweiten Abkühlschritt mit der Abkühlrate T2

umfasst und wobei der Betrag der Abkühlrate T2 kleiner als der Betrag der Abkühlrate T1 ist.

Europäisches
Patentamt
European
Patent Office
Office européen
des brevets

**EUROPÄISCHER RECHERCHENBERICHT**

Nummer der Anmeldung

**EP 21 19 1535**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2018/115529 A1 (IVOCLAR VIVADENT AG [LI]) 28. Juni 2018 (2018-06-28) | 1,4,6, 8-10, 17-22 | INV. B32B18/00 C04B35/486 |
| Y | * Anspruch 1; Beispiele 1,2 * | 11-16 | C04B35/64 A61C13/00 |
| | ----- | | |
| X | US 2021/093517 A1 (HAUPTMANN HOLGER [DE] ET AL) 1. April 2021 (2021-04-01) | 1,3,4, 6-10, 17-22 | |
| Y | * Absätze [0062] – [0098], [0139] – [0152], [0235]; Abbildung 1 * | 11-16 | |
| | ----- | | |
| X | US 2007/023971 A1 (SAHA SUBRATA [US] ET AL) 1. Februar 2007 (2007-02-01) | 1,2,6,7, 9,10, 17-22 | |
| Y | * Absätze [0002] – [0004], [0169]; Ansprüche 9-12; Abbildung 20 * | 11-16 | |
| | ----- | | |
| X | CN 112 939 598 A (SICHUAN HUIGU ZIRCONIUM IND CO LTD) 11. Juni 2021 (2021-06-11) | 1-5, 8-10,17, 18,21,22 | |
| Y | * Absatz [0002]; Beispiele 1-3,comp. ex. 1 * | 11-16 | RECHERCHIERTE SACHGEBIETE (IPC) |
| | ----- | | B32B C04B A61C |
| Y | US 2020/170763 A1 (JIANG BO [CH] ET AL) 4. Juni 2020 (2020-06-04) * Absatz [0083] – Absatz [0085] * | 11-16 | |
| | ----- | | |

Der vorliegende Recherchenbericht wurde für alle Patentansprüche erstellt

| Recherchenort | Abschlußdatum der Recherche | Prüfer |
|---|---|---|
| **Den Haag** | **13. Januar 2022** | **Raming, Tomas** |

EPO FORM 1503 03.82 (P04C03)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................
& : Mitglied der gleichen Patentfamilie, übereinstimmendes Dokument

**ANHANG ZUM EUROPÄISCHEN RECHERCHENBERICHT
ÜBER DIE EUROPÄISCHE PATENTANMELDUNG NR.**

EP 21 19 1535

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-01-2022

| Im Recherchenbericht angeführtes Patentdokument | Datum der Veröffentlichung | Mitglied(er) der Patentfamilie | Datum der Veröffentlichung |
|---|---|---|---|
| WO 2018115529 A1 | 28-06-2018 | CN 109937140 A | 25-06-2019 |
| | | EP 3558671 A1 | 30-10-2019 |
| | | JP 6893556 B2 | 23-06-2021 |
| | | JP 2020500191 A | 09-01-2020 |
| | | JP 2021152028 A | 30-09-2021 |
| | | KR 20190100236 A | 28-08-2019 |
| | | US 2019381769 A1 | 19-12-2019 |
| | | WO 2018115529 A1 | 28-06-2018 |
| US 2021093517 A1 | 01-04-2021 | EP 3759061 A1 | 06-01-2021 |
| | | US 2021093517 A1 | 01-04-2021 |
| | | WO 2019166938 A1 | 06-09-2019 |
| US 2007023971 A1 | 01-02-2007 | KEINE | |
| CN 112939598 A | 11-06-2021 | KEINE | |
| US 2020170763 A1 | 04-06-2020 | CN 111233469 A | 05-06-2020 |
| | | EP 3659574 A1 | 03-06-2020 |
| | | JP 2020083894 A | 04-06-2020 |
| | | KR 20200066226 A | 09-06-2020 |
| | | US 2020170763 A1 | 04-06-2020 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2098188 A1
- EP 2101133 A1
- WO 2012057829 A2
- WO 2015091744 A1
- WO 2015121364 A1
- WO 2011020688 A1
- EP 2703760 A1
- WO 2017189414 A1
- EP 3659548 A1

**In der Beschreibung aufgeführte Nicht-Patentliteratur**

- **M. I. MENDELSON.** *J. Am. Ceram. Soc.,* 1969, vol. 52 (8), 443-446